(19) Europäisches Patentamt
European Patent Office
Office européen des brevets

(11) Veröffentlichungsnummer : **0 183 194**
**B1**

(12) **EUROPÄISCHE PATENTSCHRIFT**

(45) Veröffentlichungstag der Patentschrift : **21.10.87**

(51) Int. Cl.4 : **C 07 C143/20**, C 07 C 91/26, A 61 K 31/185, A 23 K 1/16

(21) Anmeldenummer : **85114798.3**

(22) Anmeldetag : **21.11.85**

(54) **Menadioncholinbisulfit-Addukt und Verfahren zu dessen Herstellung.**

(30) Priorität : **28.11.84 DE 3443270**

(43) Veröffentlichungstag der Anmeldung : **04.06.86 Patentblatt 86/23**

(45) Bekanntmachung des Hinweises auf die Patenterteilung : **21.10.87 Patentblatt 87/43**

(84) Benannte Vertragsstaaten : **BE CH DE FR GB IT LI NL**

(56) Entgegenhaltungen :
**CH-A- 260 991**
**DE-B- 1 033 017**
**GB-A- 771 180**

(73) Patentinhaber : **BASF Aktiengesellschaft**
**Carl-Bosch-Strasse 38**
**D-6700 Ludwigshafen (DE)**

(72) Erfinder : **Schneider, Joachim U., Dr.**
**Plauserstrasse 17**
**D-6719 Weisenheim (DE)**
Erfinder : **Kiefer, Hans, Dr.**
**Im Sandgarten 5**
**D-6706 Wachenheim (DE)**

**EP 0 183 194 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Bisulfitaddukt von Menadion, das Vitamin K-Aktivität zeigt, die Herstellung dieser Verbindung und seine Verwendung in der Tierernährung und als Therapeutikum in der Human- und Veterinärmedizin.

Es ist schon lange bekannt, daß 2-Methyl-naphthochinon-1,4 (Menadion) eine hohe antihämorrhagische Wirkung besitzt. Menadion zählt man daher zu den Vitaminen der K-Reihe. In der Literatur wird Menadion überwiegend als Vitamin-$K_3$ bezeichnet (vgl. Ullmanns Encyklopädie der technischen Chemie, 4. Auflage, Band 23, S. 649 ff, Verlag Chemie, Weinheim, 1983).

Trotz der hervorragenden antihärmorrhagischen Wirkung des Menadions hat dieses Produkt selbst weder in der Humanmedizin noch in der Tierernährung wirtschaftliche Bedeutung erlangt. Die Gründe dafür liegen in der Unlöslichkeit des Menadions in Wasser, seiner geringen Stabilisät gegen Umgebungseinflüsse (Hitze, Licht) sowie der hautreizenden und zu Entzündungen führenden Eigenschaften des Menadions.

Produkte, die eine gleichgute antihämorrhagische Wirkung wie das Menadion besitzen und die die geschilderten Nachteile nicht bzw. weniger stark aufweisen, stellen die Additionsverbindungen von Alkali- bzw. Erdalkalihydrosulfiten an Menadion dar (u. a. DE-AS 10 33 017, FR 996 080, US 2 331 808, GB 771 180). Diese Produkte, wie z. B. das Additionsprodukt von Natriumhydrogensulfit an Menadion, in der Industrie allgemein als MSB (Menadionsodiumbisulfit) bezeichnet, haben in der Futermittelindustrie große wirtschaftliche Bedeutung erlangt.

Wissenschaftliche Untersuchungen (vgl. z. B. M. Carmack, M. B. Moore und M. E. Balis, J. Amer. Chem. Soc. 72, 844-847 (1950) und J. C. Vire, G. J. Patrlavche und G. D. Christian, Analytical Chemistry 51, (1979); S. 752 ff) sowie Erfahrungen in der Futtermittelindustrie (J. Nir. J. Kafvi und R. Cohen, Poultry Science, Vol. 57 (1978), S. 206-209) zeigen, daß selbst MSB als das am meisten eingesetzte Bisulfit-Addukt keine ausreichende Stabilität gegenüber Licht, Hitze und Feuchtigkeit besitzt.

Aus diesem Grunde hat man auch schon versucht, die Stabilität des MSB dadurch zu erhöhen, daß man es durch Reaktion mit geeigneten Salzen von schwachen Basen in schlechter wasser-lösliche Produkte überführt.

Von diesen Verbindungen hat das Menadionpyrimidinolbisulfit (MPB) die größte Verbreitung gefunden (vgl. US 3 328 169). Dieses Addukt enthält Menadion in einer Menge von 45,6 %, daneben jedoch auch 32,6 % Dimethylpyrimidinol. Die in der Tat zu beobachtende höhrere Stabilität dieser Verbindung wird jedoch durch die Anwesenheit einer körperfremden Substanz und einer beabsichtigten, geringeren Wasserlöslichkeit erkauft.

Nach den Angaben der DE-OS 28 55 851 hat man daher versucht, die stabilisierende Wirkung des körperfremden Dimethylpyrimidinols durch bestimmte Vitamine wie Vitamin $B_1$ oder $B_5$ (Nikotinsäure bzw. Nikotinamid) zu ersetzen. Diese Vitamin-Addukte sind vom Standpunkt der Physiologie sicher unbedenklicher; sie weisen jedoch noch die folgenden schwerwiegenden Nachteile auf:

Ähnlich wie MPB auch, sind alle in der DE-OS 28 55 851 beschriebenen Addukte in Wasser wesentlich schlechter löslich als MSB, und letzten Endes beruhen alle Herstellverfahren sowohl von MPB als auch der in der DE-OS 28 55 851 genannten Addukte auf der wesentlich geringeren Löslichkeit der Verfahrensprodukte im Vergleich zu der des MSB. Im Vergleich zu der ursprünglich angestrebten Überführung des Menadions in ein gut wasserlösliches Derivat (s. o.), stelen diese Produkte demnach einen Rückschritt dar.

Zum anderen besitzen die in der DE-OS 28 55 851 genannten Verbindungen nicht nur eine antihämorrhagische Wirkung, sondern auch eine ausgeprägte Wirkung des zur Stabilisierung eingesetzten Vitamins (vgl. hierzu DE-OS 28 55 851, Seite 7, 3. Abs.). Beide Wirkungen sind unlösbar in den beanspruchten Verbindungen vereint. Soll beispielsweise die Additionsverbindung der Menadionsulfonsäure an Nikotinsäure zur Vorbeugung oder zur Heilung von Hämorrhagien appliziert werden, wird zwangsläufig auch die physiologisch ganz anders wirksame Nikotinsäure zugeführt und umgekehrt.

Der Erfindung lag daher die Aufgabe zugrunde, ein Menadion-Addukt zu finden, das eine hohe antihämoorhagische Wirkung zeigt, gut wasserlöslich ist, im Vergleich zum MSB eine wesentlich höhere Stabilität besitzt, keine körperfremde Substanz enthält und keine zusätzliche unerwünschte fremde Vitamin-Wirkung aufweist. Weiterhin sollte ein Verfahren zur Herstellung dieses Adduktes gefunden werden.

Demgemäß wurde das Menadioncholinbisulfit-Addukt der Summenformel $C_{16}H_{23}O_6NS$ und der Strukturformel I

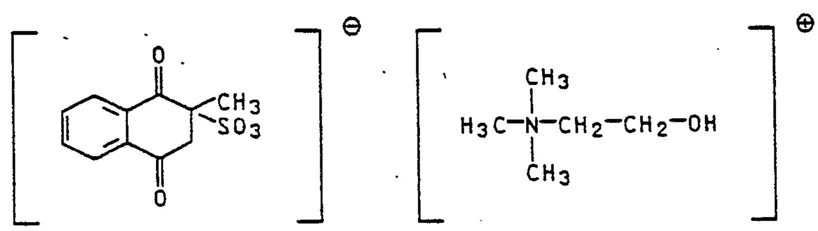

(I)

gefunden, das sich herstellen läßt, indem man Menadion mit Cholinhydrogensulfit in wäßriger Lösung umsetzt und das Reaktionsprodukt durch Kristallisation gewinnt.

Das in der Literatur bisher nicht beschriebene Additionsprodukt von Cholinhydrogensulfit an Menadion (MCB) zeigt eine gleichgute antihämorrhagische Wirkung wie MSB und eine weitaus stärkere Löslichkeit in Wasser. Beispielsweise kann man bei Zimmertemperatur stabile und gut rührbare Lösungen von MCB in Wasser mit einem MCB-Gehalt von über 70 % herstellen, während die Löslichkeit von MSB bei rund 40 % und die von MPB nur 3 % beträgt.

Wäßrige Lösungen von MCB zeigen auch nach monatelanger Lagerung bei Zimmertemperatur keinen Abfall der Vitamin-K-Wirkung. Im Vergleich dazu ist bei frisch hergestellten MSB-Lösungen nach einer einmonatigen Lagerzeit bei 20 °C bereits ein Aktivitätsverlust von 10 bis 15 % zu verzeichnen.

Besonders überraschend ist, daß MCB aus der Luft keine Feuchtigkeit anzieht und nicht wie die meisten Cholinsalze an der Luft zerfließt.

Das erfindungsgemäße Produkt wird durch Umsetzung von Cholinhydrogensulfit mit Menadion gemäß folgender Reaktionsgleichung erhalten :

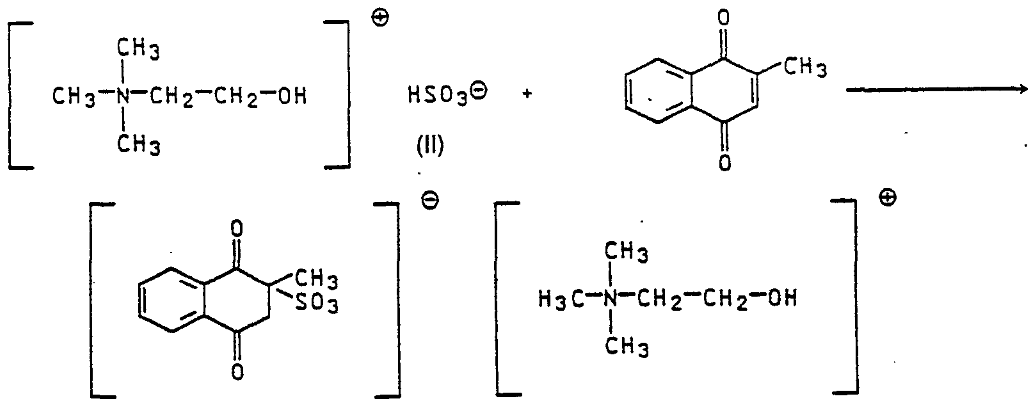
(II)

(I)

Das Cholinhydrogensulfit der Formel (II) wird zweckmäßigerweise in situ hergestellt, indem man z. B. eine käufliche, konzentrierte, wäßrige Cholinhydrogencarbonatlösung mit flüssigem oder gasförmigen Schwefeldioxid reagieren läßt, bis der pH-Wert auf 4 bis 5 abgefallen ist. Im allgemeinen wird die Cholinhydrogencarbonatlösung in einer Konzentration von 70 bis 80 % und in einer Menge von 1,0 bis 1,1 mol pro Mol Menadion verwendet.

Das Cholinhydrogensulfit wird mit feingepulvertem Menadion bei Temperaturen von 0 bis 80, vorzugsweise 40 bis 50 °C umgesetzt. Bevorzugt werden äquimolare Mengenverhältnisse beider Komponenten gewählt oder 1 bis 1,1 mol Cholinhydrogensulfit pro Mol Menadion.

Der Ablauf der Reaktion läßt sich direkt verfolgen, da das Menadion in Wasser unlöslich ist, während das Reaktionsprodukt MCB gut wasserlöslich ist. Nachdem das Menadion vollständig in Form des Menadioncholinbisulfit-Adduktes (MCB) in Lösung gegangen ist, in der Regel nach 3 bis 5 Stunden, isoliert man das MCB aus dem Reaktionsgemisch vorzugsweise in der Art, daß man bei Raumtemperatur ein Lösungsmittel zusetzt, das mit Wasser mischbar ist, die Löslichkeit des MCB in Wasser herabsetzt und in dem das MCB nicht löslich bzw. schwerlöslich ist, so daß es auskristallisiert.

Als Lösungsmittel, die zu einer guten und weitgehenden Kristallisation des MCB führen, kommen z. B. Alkohole mit 3 Kohlenstoffatomen und mit $H_2O$ mischbare Ether wie z. B. THF oder Dioxan, in Betracht. Besonders geeignet ist Isopropanol, das in der Regel in einer Menge von 50 bis 500, insbesondere 200 bis 300 Gew.%, bezogen auf das rohe Reaktionsgemisch, zugesetzt wird. Zur Vervollständigung der Kristallisation wird das Reaktionsgemisch z. B. auf 0 °C abgekühlt.

Anschließend werden die abgeschiedenen Kristalle in an sich bekannter Weise z. B. durch Filtration von der Lösung abgetrennt und getrocknet.

Das nach den erfindungsgemäßen Verfahren in hoher Ausbeute und Reinheit gewonnene Menadioncholinbisulfitaddukt ist frei von Kristallwasser, zeigt eine hervorragende Löslichkeit in Wasser und führt in wäßriger Lösung zu einem pH-Wert von 4,5-5,5. Der Prozentsatz an Menadion läßt sich gemäß dem in « USA Pharmacopeia », 15th Edition, Seite 394 beschriebenen Verfahren ermitteln, danach liegt der Menadiongehalt bei 47 bis 48 % (Theorie : 48,2 %). Fürden Einsatz in pharmazeutischen Zubereitungen läßt sich MCB durch Umkristallisieren aus wasserfreien niederen Alkoholen noch weiter reinigen.

Aufgrund seiner guten Eingenschaften wie hohe Reinheit, hervorragende Wasserlöslichkeit und hohe Stabilität, die mit dem physiologisch völlig unbedenklichen Baustein Cholin erreicht wird, ist das MCB ausgezeichnet für eine Verwendung im human- und veterinärmedizinischen Bereich als antihämorrhagisches Mittel geeignet.

Die Löslichkeit des MCB ist so hoch, daß sich isotonische Lösungen ohne Zusatz von Kochsalz herstellen lassen.

Pharmazeutische Zubereitungen, die als wesentlichen Wirkstoff MCB enthalten, können z. B.

Elixiere, Salben, Kapseln, Tabletten oder sterile, isotonische wäßrige Lösungen sein. Die Dosierung wird im allgemeinen in der Größenordnung liegen wie sie für bisher übliche Menadion-Verbindungen gebräuchlich ist.

Weiterhin eignet sich MCB hervorragend zum Einsatz in der Tierernährung, z. B. als Vitamin-Zusatz zu Futtermitteln oder Tränkflüssigkeiten zur Prophylaxe von Hämorrhagien.

Zur Verbesserung der Rieselfähigkeit bzw. des Fließverhaltens kann festes MCB hierzu mit bekannten Fließhilfsmitteln wie z. B. mit speziellen Kieselsäuren, Phosphaten oder auch Calciumsalzen von Fettsäuren versetzt werden.

Die Menge an MCB wird im allgemeinen bei 0,1 bis 5 mg pro kg Futtermittel liegen. Zweckmäßigerweise wird zunächst dem Bedarf der jeweiligen Tierart angepaßt, eine entsprechende Vitaminvormischung, bestehend aus Vitaminen und einem Träger hergstellt. In diesem Wirkstoffkonzentrat liegt MCB in einer Menge von 5 bis 50, insbesondere von 10 bis 30 g/kg Konzentrat vor. Dieses Wirkstoffkonzentrat wird mit anderen Futtermittelkomponenten zu einem Prämix vermischt, aus dem in einer weiteren Abmischung das eigentliche Futtermittel hergestellt wird. Eine typische Vitamin-Vormischung ist in Beispiel 3 angegeben.

In Tränkflüssigkeiten ist MCB in der Regel in einer Menge von 1 bis 5 mg pro Liter enthalten. Zubereitungen für Tränkflüssigkeiten enthalten MCB in Mengen von 0,5-2,5 %.

## Beispiel 1

Unter ständigem Rühren leitet man in 363 g einer 75 %igen wäßrigen Cholinhydrogencarbonatlösung solange gasförmiges Schwefeldioxid ein, bis ein pH-Wert von 4,5 erreicht ist. Danach trägt man unter fortwährendem Rühren bei einer Temperatur von 40 bis 45 °C 258 g Menadion in feingepulverter Form ein. Im Verlaufe von 4 Stunden geht das Menadion vollständig in Lösung. Nach der Zugabe von 1 500 ml Isopropanol kühlt man das Reaktionsgemisch im Verlauf von 3 Stunden auf 0 °C ab und filtriert die abgeschiedenen Kristalle über eine Fritte ab. Nach dem Waschen des Kristallisates mit 300 ml Isopropanol trocknet man das Kristallisat auf der Fritte durch Durchblasen von trockener Luft oder trockenem Stickstoff. Man erhält 465 g praktisch farblose Kristalle vom Schmp. 128 °C mit einem Menadion-Gehalt (nach USA-Pharmakopeia) von 47,5 % (Theorie : 48,2 %). Ausbeute : 85,6 % d. Th. (analytisch wiedergefundenes zu eingesetztem Menadion).

$C_{16}H_{23}O_6$ NS (MG 357)
ber. : C 53,8 %, H 6,4 %, O 26,9 %, N 3,9 %, S 8,9 %
gef. : C 53,5 %, H 6,4 %, O 26,6 %, N 4,0 %, S 8,9 %

Das NMR-Spektrum steht vollständig in Einklang mit der weiter vorne angegebenen Struktur.

## Beispiel 2

Jeweils 20 %ige Lösungen von käuflichem MSB und von nach Beispiel 1 erhältlichem MCB in $D_2O$ werden 6 Stunden auf 80 °C erhitzt. Vor und nach dem Erhitzen werden die NMR-Spektren der beiden Lösungen aufgenommen. Dabei stellt man fest, daß in der erhitzten Probe im Falle des MCB keine neuen NMR-Signale auftreten und die alten Linien keine Intensitätsveränderungen erfahren.

Unter den gleichen Bedingungen gehen der NMR-analytischen Auswertung zufolge 36 % des MSB in die isomere, Vitamin-K-unwirksame Verbindung über (vgl. hierzu J. Amer. Chem. Soc. 65, 1943, 1210).

## Beispiel 3

Vitamin-Vormischung

| | |
|---|---|
| Vitamin A 500 000 IE/g | 144,7 kg |
| Vitamin $D_3$ 500 000 IE/g | 18,1 kg |
| Vitamin E 50 % | 160,8 kg |
| Vitamin $B_1$ | 9,3 kg |
| Vitamin $B_2$ | 74,5 kg |
| Vitamin $B_6$ | 12,4 kg |
| Vitamin $B_{12}$ (2 %) | 2,0 kg |
| Vitamin C | 120,6 kg |
| Vitamin $K_3$ als MCB | 12,1 kg |
| Folsäure | 3,0 kg |
| Nikotinsäure | 120,6 kg |
| Ca-D-Pantothenat | 63,4 kg |
| Sojaöl | 10,0 kg |
| Weizengrießkleie (Träger) | 248,5 kg |
| | 1 000,0 kg |

**Patentansprüche**

1. Menadioncholinbisulfit-Addukt der Formel I

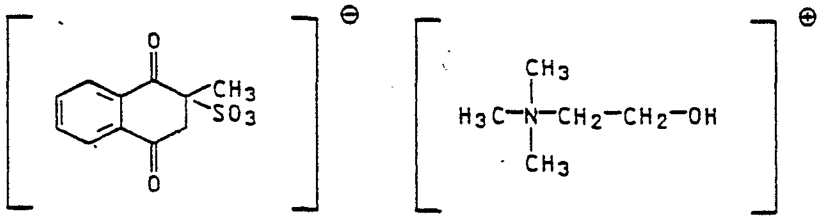

(I)

2. Verfahren zur Herstellung von Menadioncholinbisulfit-Addukt gemäß Anspruch 1, dadurch gekennzeichnet, daß man Menadion mit Cholinhydrogensulfit in wäßriger Lösung umsetzt und das Reaktionsprodukt durch Kristallisation gewinnt.

3. Verfahren nach Anspruch 2, dadurch gekennzeichnet, daß man das Cholinhydrogensulfit in situ aus konzentrierter, wäßriger Cholinhydrogencarbonatlösung und Schwefeldioxid herstellt.

4. Verfahren nach Anspruch 2, dadurch gekennzeichnet, daß man die Umsetzung bei Temperaturen von 0 bis 80 °C durchführt.

5. Verfahren nach Anspruch 2, dadurch gekennzeichnet, daß man nach der Umsetzung ein mit Wasser mischbares Lösungsmittel zusetzt, das die Löslichkeit des Menadioncholinbisulfit-Adduktes in Wasser herabsetzt und in dem das Addukt nicht löslich bzw. schwerlöslich ist.

6. Verfahren nach Anspruch 5, dadurch gekennzeichnet, daß man nach der Umsetzung Isopropanol in einer Menge von 50 bis 500 Gew.%, bezogen auf das rohe Reaktionsgemisch, zusetzt.

7. Menadioncholinbisulfit-Addukt gemäß Anspruch 1 zur Verwendung als antihämorrhagisches Mittel.

8. Futtermittel oder Tränkflüssigkeit enthaltend geringe Mengen an Menadioncholinbisulfit-Addukt gemäß Anspruch 1 zur Prophylaxe von Hämorrhagien.

**Claims**

1. The menadione choline bisulfite adduct of the formula I

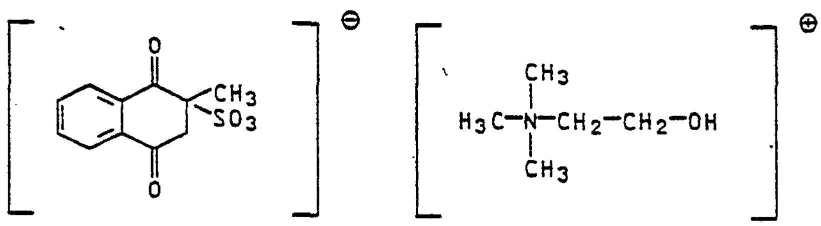

(I)

2. A process for the preparation of the menadione choline bisulfite adduct as claimed in claim 1, wherein menadione is reacted with choline bisulfite in aqueous solution, and the reaction product is isolated by crystallization.

3. A process as claimed in claim 2, wherein the choline bisulfite is prepared in situ from concentrated, aqueous choline bicarbonate solution and sulfur dioxide.

4. A process as claimed in claim 2, wherein the reaction is carried out at from 0 to 80 °C.

5. A process as claimed in claim 2, wherein a water-miscible solvent which reduces the solubility of the menadione choline bisulfite adduct in water and in which the adduct is insoluble or sparingly soluble is added after the reaction is over.

6. A process as claimed in claim 5, wherein, after the reaction is over, isopropanol is addied in an amount of from 50 to 500 % by weight, based on the crude reaction mixture.

7. The menadione choline bisulfite adduct as claimed in claim 1 for use as an antihemorrhagic agent.

8. An animal feed or drinking liquid containing small amounts of the menadione choline bisulfite adduct as claimed in claim 1 for the prophylactic treatment of hemorrhages.

**Revendications**

1. Produit d'addition du type bisulfite de ménadioncholine de la formule I

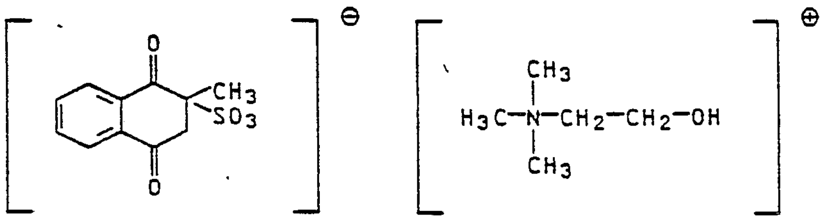 (I)

2. Procédé de préparation du produit d'addition du type bisulfite de ménadioncholine suivant la revendication 1, caractérisé en ce que l'on fait réagir le ménadion sur l'hydrogénosulfite de choline en solution aqueuse et on récupère le produit de la réaction par cristallisation.

3. Procédé suivant la revendication 2, caractérisé en ce que l'on prépare l'hydrogénosulfite de choline in situ à partir d'une solution aqueuse concentrée d'hydrogénocarbonate de choline et d'anhydride sulfureux.

4. Procédé suivant la revendication 2, caractérisé en ce que l'on réalise la réaction à des températures de 0 à 80 °C.

5. Procédé suivant la revendication 2, caractérisé en ce qu'après la réaction, on ajoute un solvant miscible à l'eau qui réduit la solubilité du produit d'addition du type bisulfite de ménadioncholine dans de l'eau et dans lequel le produit d'addition n'est pas soluble ou seulement difficilement soluble.

6. Procédé suivant la revendication 5, caractérisé en ce qu'après la réaction, on ajoute de l'isopropanol en une proportion de 50 à 500 % en poids, par rapport au mélange réactionnel brut.

7. Produit d'addition du type bisulfite de ménadioncholine suivant la revendication 1 en vue de son utilisation comme agent anto-hémorragique.

8. Nourriture ou liquide de boisson contenant de faibles quantités d'un produit d'addition du type bisulfite de ménadioncholine suivant la revendication 1, en vue de la prophylaxie d'hémorragies.